(19) Europäisches Patentamt
European Patent Office
Office européen des brevets

(11) EP 0 619 308 B1

(12) **FASCICULE DE BREVET EUROPEEN**

(45) Date de publication et mention de la délivrance du brevet:
**11.03.1998 Bulletin 1998/11**

(51) Int Cl.$^6$: **C07D 213/73**, G02F 1/35

(21) Numéro de dépôt: **94400743.4**

(22) Date de dépôt: **06.04.1994**

(54) **Structures cristallines d'halogénures de 2-amino-5-nitropyridinium, procédé de préparation desdites structures cristallines, et dispositif à effet électrooptique comportant de telles structures**

Kristalline Strukturen aus halogeniden von 2-Amino-5-Nitro-Pyridinium, Verfahren zu ihrer Herstellung und eine elektrooptische Vorrichtung mit diesen Strukturen

Crystalline structures of halides of 2-amino-5-nitro-pyridinium, process for their preparation and an electrooptical device with such structures

(84) Etats contractants désignés:
**DE GB**

(30) Priorité: **07.04.1993 FR 9304116**

(43) Date de publication de la demande:
**12.10.1994 Bulletin 1994/41**

(73) Titulaires:
- **FRANCE TELECOM**
  **75015 Paris (FR)**
- **CENTRE NATIONAL DE LA RECHERCHE SCIENTIFIQUE (CNRS)**
  **75794 Paris Cédex 16 (FR)**

(72) Inventeurs:
- **Pecaut, Jacques**
  **F-84000 Avignon (FR)**
- **Levy, Jean-Pierre**
  **F-38100 Grenoble (FR)**
- **Masse, René**
  **F-38290 Crolles (FR)**
- **Zyss, Joseph**
  **F-92330 Sceaux (FR)**
- **Hierle, Rolland**
  **F-75013 Paris (FR)**

(74) Mandataire: **Ahner, Francis**
**CABINET REGIMBEAU**
**26, avenue Kléber**
**75116 Paris (FR)**

(56) Documents cités:
**EP-A- 0 488 869**

- **JOURNAL OF THE CHEMICAL SOCIETY, CHEMICAL COMMUNICATIONS no. 23 , 1989 , LETCHWORTH GB pages 1856 - 1859 C.B. AAKERÖY ET AL. 'A novel class of salts for harmonic generation'**
- **ACTA CRYSTALLOGRAPHICA SECTION B: STRUCTURAL SCIENCE vol. B49, no. 2 , 1 Avril 1993 , COPENHAGEN pages 277 - 282 J. PECAUT; R. MASSE 'Structure of Bis(2-amino-5-nitropyridinium) dichromate...'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 619 308 B1

## Description

La présente invention concerne de nouvelles structures cristallines utiles en optique non-linéaire, et en électrooptique. Elle concerne plus particulièrement de nouvelles structures cristallines présentant des effets non linéaires quadratiques et une plage de transparence large s'étendant du visible au proche infrarouge. Elle concerne enfin un procédé de préparation de ces structures cristallines et un dispositif à effet électrooptique comportant une telle structure cristalline.

De part sa structure non centrosymétrique qui lui confère un tenseur de susceptibilité non-linéaire quadratique $\chi^2$, le cristal selon l'invention est exploitable dans toute configuration de mélange à trois ondes de type "tout-optique" (génération de second-harmonique, conversion de fréquences, effets paramétriques) et électrooptique (effet Pockels).

La recherche de structures cristallines présentant des effets non linéaires quadratiques accrus par rapport aux matériaux tels que le dihydrogéno-phosphate de potassium (Okada et al. Jap. J. Appl. Phys. 16, 55, 1977) ainsi qu'une plage de transparence large s'étendant du visible au proche infrarouge s'est orientée au cours des dix dernières années vers les molécules organiques telles qu'elles ont été répertoriées par exemple dans le livre édité par D.S. Chemia et J. Zyss, Academic Press (1987) "Nonlinear Optical Properties of Organic Molecules and Crystals".

Ces cristaux organiques peuvent se présenter comme un ensemble de dipoles moléculaires, constituant un empilement polaire dans la maille cristalline.

Le coefficient non linéaire le plus élevé est orienté le long de ces dipoles. Parmi les structures cristallines de ce type, on peut citer :

- le POM (3-méthyl-4-nitropyridine-1-oxyde) (J. Zyss, D.S. Chemia et J.F. Nicoud J. Chem. Phys. 74, 4800, (1981)),
- le NPP (N-(4-nitrophenyl)-L-prolinol) (J. Zyss, J.F. Nicoud et A. Coquillay, J. Chem. Phys. 81, 4160 (1984)),
- le NPAN (N-(4-nitrophenyl)-N-dimethylamino-aceto-nitrile) (P. Vidakovic, M. Coquillay et A. Salin, J. Opt. Soc. Am. B4, 998, (1987)).

Ces cristaux présentent une non-linéarité exceptionnellement élevée (par exemple $d_{21}$ de l'ordre de 50 pm/V pour le NPP) au prix toutefois d'une transparence limitée vers la partie bleue du spectre. Par ailleurs, la cohésion et la stabilité à l'air de tels cristaux dans des conditions usuelles d'application peut pour certains d'entre eux présenter des inconvénients : c'est en particulier le cas du POM qui doit être immergé dans un liquide protecteur et présente par ailleurs une tendance marquée à la sublimation.

Il a récemment été proposée par R. Masse et J. Zyss (Molecular Engineering 1, 141, 1991) d'encapsuler des molécules organiques non-linéaires dans un réseau "charpente" constitué d'anions minéraux associés entre eux par liaison hydrogène simple ou multiple, aux anions minéraux. Les polyanions proposés sont de type arséniate $(H_2AsO_4^-)_n$ sulfate $(HSO_4^-)_n$ ou phosphate $(H_2PO_4^-)_n$. Un cristal particulièrement intéressant a été identifié dans cette famille le 2-amino-5-nitropyridinium dihydrogène monophosphate (2A5NPDP).

Cette structure cristalline, de symétrie ponctuelle mm2 présente une inclinaison de l'ordre de 36° des dipôles de transition de l'entité organique par rapport à l'axe polaire cristallin, ce qui favorise le coefficient $d_{31}$ (de l'ordre de 10 pm/V) et les effets paramétriques exigeant l'accord de phase par biréfringence (génération de second-harmonique, amplification paramétrique).

Il est néanmoins souhaitable de trouver d'autres structures cristallines où un coefficient $d_{ii}$ (avec i=1,2 ou 3) serait préférentiellement optimisé par l'alignement le plus parfait possible des molécules dans la maille le long d'une direction cristallographique i à l'image des cristaux de MNA (2-méthyl-4-nitroaniline) comme ils ont été décrits par G. Lipscomb, A. Garito et R. Narang (J. Chem. Phys. 75, 1509, (1981)) ou des cristaux de DMACB (4,4'-diméthylaminocyanobiphényl) comme ils ont été décrits par J.Zyss, I. Ledoux, M. Bertault, et E. Toupet (Chem. Phys. 150, 125, (1991).

On rappelle que les coefficients $d_{21}$ et $d_{22}$ se rapportent aux coefficients du tenseur de susceptibilité non-linéaire quadratique, l'axe 2 étant l'axe de symétrie linéaire dans le groupe cristallographique $P2_1$ (rotation contrastée).

On signalera, par ailleurs, G.R. Meredith (ACS Symposium Series, 233, 27-56 (1983)) qui a développé des sels organiques pour l'optique non-linéaire quadratique, dans lesquels les cations présentant un caractère de transfert de charge intra-moléculaire sont associés à des anions organiques chiraux, achiraux ou minéraux. Les anions utilisés dans cette référence sont des unités indépendantes qui ne s'organisent pas en une structure polyanionique et la cohésion mécanique de telles structures n'est pas encore suffisante.

Au vu de l'art antérieur exposé ci-dessus, un des objets de l'invention est de proposer de nouvelles structures cristallines non centro-symétriques, présentant des effets non-linéaires quadratiques.

Un second objet de l'invention est de proposer des structures cristallines présentant des effets non-linéaires quadratiques accrus par rapport à ceux de l'art antérieur.

Un autre objet de la présente invention est de proposer des structures cristallines présentant des effets non-linéaires quadratiques et une excellente cohésion des entités moléculaires organiques dans le cristal.

D'autres objets de l'invention apparaîtront à la lecture de la description qui va suivre.

Selon la présente invention, la structure cristalline non centro-symétrique est constituée d'halogénure de 2-amino-5-nitropyridinium, éventuellement substitué.

Les substituants des cations 2-amino-5-nitropyridinium sont ceux qui ne modifient pas de façon significative l'orientation favorable de l'entité organique polarisable dans la maille cristalline.

L'halogénure est de préférence un ion chlorure, un ion bromure ou un ion fluorure.

Selon une variante préférée, la structure cristalline est caractérisée en ce que le cation répond à la formule suivante :

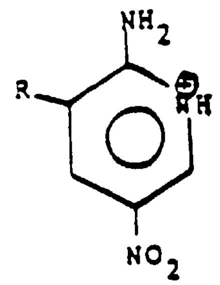

dans laquelle :

R représente un atome d'hydrogène, un radical méthyle ou éthyle, un radical hydroxy.

De préférence encore, le cation est le 2-amino-5-nitropyridinium.

Sans être lié de quelque façon que ce soit à une théorie scientifique, le déposant pense néanmoins que ces structures possèdent une cohésion spécifique due à des liaisons hydrogène courtes donc très énergétiques, permettant l'agrégation des cations autour des anions halogénures. Ce mode de liaison assure à la structure cristalline selon l'invention, stabilité, transparence et facilité de croissance.

De préférence enfin, la structure cristalline selon l'invention est un mono-cristal.

L'invention concerne également un procédé de préparation des structures cristallines telles qu'elles viennent d'être décrites dans la description ci-dessus, caractérisé en ce qu'on mélange une mole de 2-amino-5-nitropyridine éventuellement substitué avec au moins une mole d'halogénure d'hydrogène dans un solvant approprié, en ce que l'on fait cristalliser le mélange et en ce que l'on récupère les structures cristallines.

Le solvant approprié est notamment de l'eau ou un mélange acétone/eau.

De préférence, on mélangera une mole de 2-amino-5-nitropyridine éventuellement substituée avec entre 0,3 et 2 moles d'halogénure d'hydrogène.

La cristallisation s'effectue par évaporation lente du solvant. Il s'agit donc d'une méthode bien connue de l'homme du métier.

Avantageusement, dans une solution saturée en rotation d'un mélange anions-cations, sera présent un germe cristallin d'une structure cristalline telle que décrite ci-dessus. L'évaporation lente conduit à la cristallisation recherchée, à température constante (ambiante notamment). On peut également abaisser progressivement cette température.

Les monocristaux possèdent les propriétés requises, c'est-à-dire une structure cristalline polaire compatible à la fois avec les exigences de l'optique non-linéaire (effets quadratiques) et de la ferroélectricité, une réponse non-linéaire et un compromis transparence-efficacité satisfaisant.

L'invention concerne enfin un dispositif à effet électrooptique comportant une structure cristalline telle que décrite précédemment. Ces dispositifs génèrent une second-harmonique ou plus généralement une somme ou différence de fréquences. Les applications qui découlent de ces propriétés avantageuses sont nombreuses tant en cristal massif, qu'en structure guidante telles que :

- modulation de phase d'une porteuse optique
- modulation d'amplitude
- coupleur directif,
- croisement électrooptique et plus généralement point de commutation électrooptique,
- échantillonnage électrooptique,
- logique électronique,
- déclenche électrooptique de lasers ("Q-Switch") sans préjudice des applications exigeant un accord de phase par biréfringence telles que :
- génération de second-harmonique,
- conversions de fréquence
- amplification, émission et oscillation paramétriques.

A titre d'exemple, on décrit ci-après une cavité laser à déclenche électrooptique munie d'une structure cristalline selon l'invention.

Dans un tel dispositif, une structure cristalline selon l'invention est introduite dans la cavité d'un laser (par exemple un laser YAG émettant à 1,06 ou 1,34 m et plus généralement tout laser émettant dans la plage de transparence du cristal de 0,45 à 1,7 m) dans une configuration de type cellule de Pockels. Le jeu de polarisations et réflexions aboutit à un blocage de l'émission lorsque le cristal est sous tension. En l'absence de tension, le cristal redevient passant et la cavité peut émettre. Un schéma général de principe est par exemple indiqué dans Hellwarth R.W. "Q Modulation of lasers" dans Lasers, vol. 1, A.K. Levine ed. (New York, Marcel Dekker, 1996, p. 253).

L'invention est maintenant illustrée par les exemples de réalisation donnés à titre indicatif ci-après et par les figures 1 et 2 en annexe.

La figure I représente la maille cristalline du cristal de chlorure de 2-amino-5-nitropyridinium projetée dans le plan cristallographique [$\vec{a}$,$\vec{c}$].

La figure 2 représente le même cristal projeté dans le plan [$\vec{a}$, $\vec{b}$].

La structure non centro-symétrique des cristaux selon l'invention apparaît à la lecture de ces figures où l'on remarque une couche d'anions séparant les dipôles organiques, orientés selon des motifs de type "herring bone" dans une direction polaire unique (structure $P2_1$).

EXEMPLE 1

On réalise la dissolution complète d'une mole de 2-amino-5-nitropyridine (2A5NP) dans le volume minimum d'acétone à la température ambiante. 30cc d'une solution molaire d'acide chlorhydrique ou bromhydrique sont ajoutés. Les solutions obtenues sont lentement évaporées à température ambiante et donnent aisément des cristaux transparents de dimensions : 4mm x 4mm x 4mm. La réaction peut aussi se faire dans l'eau à 40°C : on dissoudra alors 0.8 mole de 2A5NP pour une mole d'acide chlorhydrique ou bromhydrique. L'élévation de température facilite la dissolution de la 2-amino-5nitropyridine dans le mélange acétone-eau.

Les réactions chimiques s'écrivent :

$$C_5H_5N_3O_2 + HCl \rightarrow C_5H_6N_3O_2^+ + Cl^- \rightarrow C_5H_6N_3O_2^+ \cdot Cl^- \text{ (2A5NPCl)}$$

$$C_5H_5N_3O_2 + HBr \rightarrow C_5H_6N_3O_2^+ + Br^- \rightarrow C_5H_6N_3O_2^+ \cdot Br^- \text{ (2A5NPBr)}$$

L'étude des structures cristallines a permis sans ambiguité de déterminer les formules chimiques et l'isotypie des deux composés. Les phases actives sont parfaitement caractérisées :

2A5NPCl a=9,956(7), b=7,737(1), c=4,813(1) A béta=95,86(9)° $P2_1$
2A5NPBr a=10,074(7), b=7,805(1), c=4,949(1) A béta=95,72(9)° $P2_1$

**EXEMPLE 2 : croissance cristalline**

L'évaporation de solutions contenant une mole 2A5NP pour une mole HCl (ou HBr) à l'air, à température ambiante produit de beaux cristaux de chlorure ou de bromure de 2-amino-5-nitropyridinium ayant tous deux la même morphologie (symétrie $P2_1$). Des cristaux trapus de 7mmx7mmx5mm, voire de 10mmx6mmx5mm pour les chlorures, ont été obtenus aisément.

Ces cristaux ont des propriétés thermiques et mécaniques équivalentes à celles du 2A5NPDP.

Par ailleurs, d'après les tests sur poudre de génération de second harmonique (illumination par un laser Nd:YAC, $\lambda$ =1,06 $\mu$m) leur efficacité s'apparente à celle du POM et du NPP.

## Revendications

1. Structure cristalline non centrosymétrique constituée d'halogénure de 2-amino-5-nitropyridinium éventuellement substitué.

2. Structure cristalline selon la revendication 1, caractérisée en ce que l'halogénure est un ion chlorure, un ion bromure ou un ion fluorure.

3. Structure cristalline selon l'une des revendications 1 ou 2, caractérisée en ce que le cation répond à la formule

suivante :

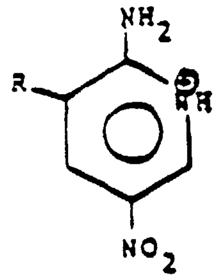

dans laquelle :
R représente un atome d'hydrogène, un radical méthyle ou éthyle, un radical hydroxy.

**4.** Structure cristalline selon l'une des revendications 1 ou 2, caractérisée en ce que le cation est le 2-amino-5-nitropyridinium.

**5.** Structure cristalline selon l'une des revendications 1 à 4, caractérisée en ce qu'elle est un monocristal.

**6.** Procédé de préparation des structures cristallines selon l'une des revendications 1 à 5, caractérisé en ce que l'on mélange une mole de 2-amino-5-nitropyridine éventuellement substitué avec entre 0,3 et 2 moles d'halogènure d'hydrogène dans un solvant approprié, en ce que l'on fait cristalliser le mélange et en ce que l'on récupère les structures cristallines.

**7.** Procédé selon la revendication 6, caractérisé en ce que le solvant est de l'eau ou un mélange acétone/eau.

**8.** Procédé de préparation selon l'une des revendications 6 ou 7, caractérisé en ce que, dans la solution saturée de mélange, un germe cristallin d'une structure cristalline selon l'une des revendications 1 à 5 est présent et en ce que la cristallisation est effectuée par évaporation du solvant à température ambiante ou par abaissement progressif de la température.

**9.** Dispositif à effet électrooptique comportant une structure cristalline selon l'une des revendications 1 à 5.

**10.** Dispositif selon la revendication 9, caractérisé en ce qu'il s'agit d'une cavité laser à déclenche électrooptique.

**11.** Dispositif pour générer une somme ou différence de fréquences, caractérisé en ce qu'il comporte une structure cristalline selon l'une des revendications 1 à 5, notamment un dispositif d'amplification, d'émission et oscillation paramétrique.

**Claims**

**1.** Noncentrosymmetric crystal structure consisting of an optionally substituted 2-amino-5-nitropyridinium halide.

**2.** Crystal structure according to Claim 1, characterized in that the halide is a chloride ion, a bromide ion or a fluoride ion.

**3.** Crystal structure according to either of Claims 1 and 2, characterized in that the cation corresponds to the following formula:

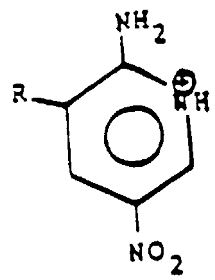

in which:
R denotes a hydrogen atom, a methyl or ethyl radical or a hydroxyl radical.

4. Crystal structure according to either of Claims 1 and 2, characterized in that the cation is 2-amino-5-nitropyridinium.

5. Crystal structure according to one of Claims 1 to 4, characterized in that it is a monocrystal.

6. Process for the preparation of the crystal structures according to one of Claims 1 to 5, characterized in that one mole of an optionally substituted 2-amino-5-nitropyridine is mixed with between 0.3 and 2 moles of a hydrogen halide in a suitable solvent, in that the mixture is caused to crystallize and in that the crystal structures are recovered.

7. Process according to Claim 6, characterized in that the solvent is water or an acetone/water mixture.

8. Process of preparation according to either of Claims 6 and 7, characterized in that a crystal seed of a crystal structure according to one of Claims 1 to 5 is present in the saturated solution of mixture and in that the crystallization is performed by evaporation of the solvent at ambient temperature or by gradual lowering of the temperature.

9. Device with an electro-optical effect comprising a crystal structure according to one of Claims 1 to 5.

10. Device according to Claim 9, characterized in that it is a laser cavity with electro-optical triggering.

11. Device for generating a sum or difference of frequencies, characterized in that it comprises a crystal structure according to one of Claims 1 to 5, especially a device for parametric amplification, emission and oscillation.

**Patentansprüche**

1. Nicht-zentrosymmetrische, kristalline Struktur, die aus einem gegebenenfalls substituierten Halogenid von 2-Amino-5-nitropyridinium besteht.

2. Kristalline Struktur nach Anspruch 1, dadurch gekennzeichnet, daß das Halogenid ein Chloridion, ein Bromidion, oder ein Fluoridion ist.

3. Kristalline Struktur nach einem der Ansprüche 1 oder 2, dadurch gekennzeichnet, daß das Kation die folgende Formel aufweist:

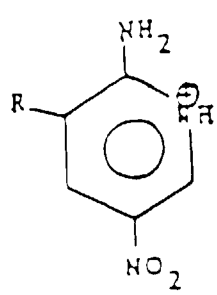

wobei
R für ein Wasserstoffatom, ein Methyl- oder ein Ethyl-Radikal, ein Hydroxyradikal steht.

4. Kristalline Struktur nach einem der Ansprüche 1 oder 2, dadurch gekennzeichnet, daß das Kation 2-Amino-5-nitropyridinium ist.

5. Kristalline Struktur nach einem der Ansprüche 1 bis 4, dadurch gekennzeichnet, daß die kristalline Struktur als Monokristall vorliegt.

6. Verfahren zur Herstellung von kristallinen Strukturen nach einem der Ansprüche 1 bis 5, dadurch gekennzeichnet, daß ein Mol von gegebenenfalls substituiertem 2-Amino-5-nitropyridin mit einer Menge von zwischen 0,3 und 2 Mol Wasserstoffhalogenid in einem geeigneten Lösungsmittel vermischt wird, wobei man das Gemisch kristallisieren läßt und die kristallinen Strukturen wiedergewonnen werden.

7. Verfahren nach Anspruch 6, dadurch gekennzeichnet, daß das Lösungsmittel Wasser oder eine Azeton/Wassermischung ist.

8. Herstellungsverfahren nach einem der Ansprüche 6 oder 7, dadurch gekennzeichnet, daß in einer mit dem Gemisch gesättigten Lösung ein Kristallkeim von einer kristallinen Struktur nach einem der Ansprüche 1 bis 5 vorliegt, und die Kristallisierung beim Abdampfen des Lösungsmittels bei Raumtemperatur oder bei einer schrittweisen Absenkung der Temperatur ausgeführt wird.

9. Vorrichtung mit elektro-optischem Effekt, die eine kristalline Struktur nach einem der Ansprüche 1 bis 5 aufweist.

10. Vorrichtung nach Anspruch 9, dadurch gekennzeichnet, daß es sich um eine Laserkavität mit elektro-optischer Auslösung handelt.

11. Vorrichtung zur Erzeugung einer Summe oder einer Differenz von Frequenzen, dadurch gekennzeichnet, daß die Vorrichtung eine kristalline Struktur nach einem der Ansprüche 1 bis 5 aufweist, insbesondere eine Vorrichtung zur parametrischen Verstärkung, Emission und Oszillation.

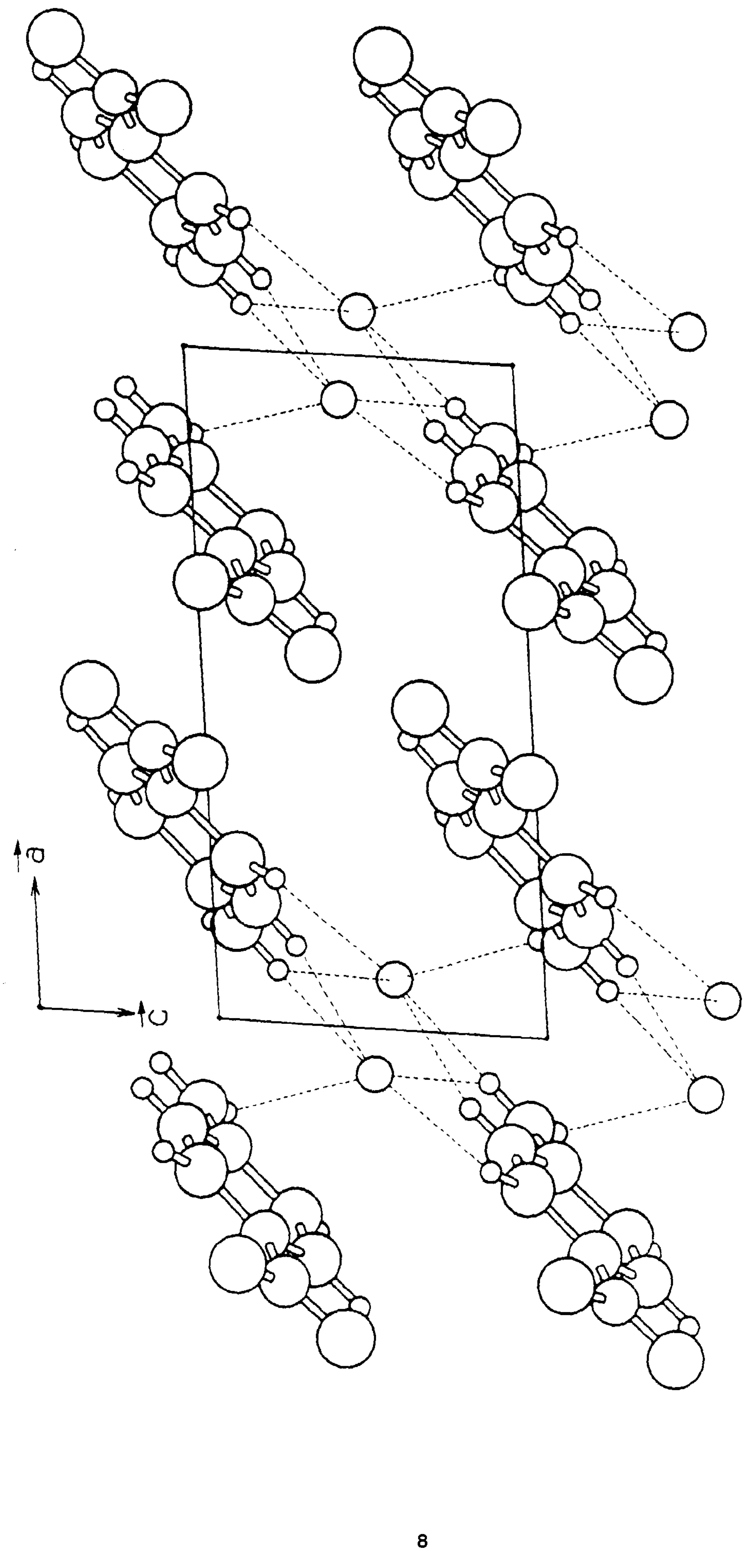


FIG. 1

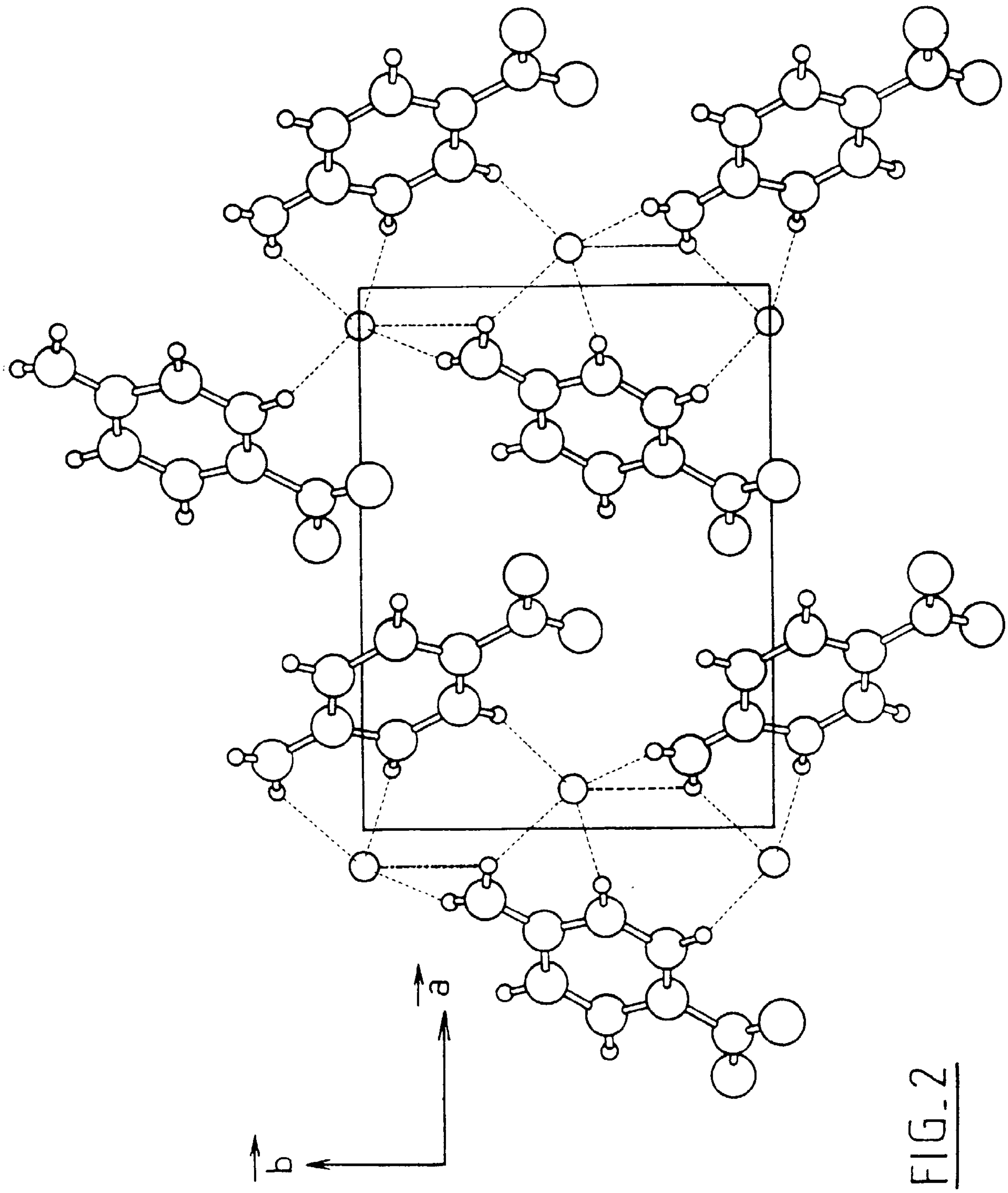


FIG_2